(19) Europäisches Patentamt
European Patent Office
Office européen des brevets

(11) EP 3 991 761 A1

(12) # EUROPEAN PATENT APPLICATION
published in accordance with Art. 153(4) EPC

(43) Date of publication:
**04.05.2022 Bulletin 2022/18**

(21) Application number: **19934518.2**

(22) Date of filing: **26.06.2019**

(51) International Patent Classification (IPC):
***A61L 27/20*** *(2006.01)* ***A61L 27/52*** *(2006.01)*
***A61F 2/00*** *(2006.01)* ***A61K 8/73*** *(2006.01)*
***A61K 8/02*** *(2006.01)* ***A61Q 19/00*** *(2006.01)*

(52) Cooperative Patent Classification (CPC):
**A61F 2/00; A61K 8/02; A61K 8/73; A61L 27/20; A61L 27/52; A61Q 19/00**

(86) International application number:
**PCT/KR2019/007709**

(87) International publication number:
**WO 2020/262727 (30.12.2020 Gazette 2020/53)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Nature Costech Co., Ltd.**
**Chungcheongbuk-do 28578 (KR)**

(72) Inventors:
- **JEON, So-Young**
**Cheongju-si Chungcheongbuk-do 28650 (KR)**
- **SONG, Woo-Young**
**Cheongju-si Chungcheongbuk-do 28374 (KR)**

(74) Representative: **Byrne, Declan**
**André Roland SA**
**P.O. Box 352**
**1000 Lausanne 22 (CH)**

(54) **DERMAL FILLER COMPOSITION COMPRISING MODIFIED CELLULOSE**

(57) The present invention relates to a dermal filler composition including modified cellulose, and more particularly, to a dermal filler composition including modified cellulose, which is prepared under specific conditions during processing cellulose and has specific physical properties, wherein the composition contains a new concept of modified cellulose which may have excellent moisturizing effect as well as skin tissue repairability thus to be useful in a dermal filler or a skin beauty enhancing composition, etc., and in particular, has excellent physical properties such as water retention value, storage modulus and loss modulus.

[FIG. 1]

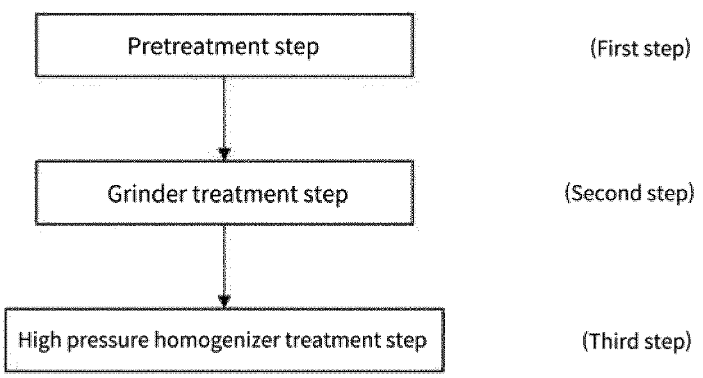


EP 3 991 761 A1

## Description

[Technical Field]

The present invention relates to a dermal filler composition including modified cellulose, and more particularly, to a dermal filler composition including modified cellulose, which is prepared under specific conditions during processing cellulose and has specific physical properties, wherein the composition contains a new concept of modified cellulose which may have excellent moisturizing effect as well as skin tissue repairability thus to be used in a dermal filler or a skin beauty enhancing composition, etc., and in particular, has excellent physical properties such as water retention value (WRV), storage modulus and loss modulus.

[Background Art]

Fiber cellulose constituting natural wood has many restrictions on use in its natural state. However, when processing into a smaller size, a specific surface area of the cellulose is greatly increased, and thereby new physical properties may be expected. As known in the art, studies conducted by ITT Rayonier laboratory have converted cellulose into a gel-type material by processing cellulose at high temperature and high pressure using a Gaulin-type high pressure homogenizer, which is a milk processing device.

After the above experiment, a mechanical method for processing cellulose into a nano size has been continuously studied, and depending on driving methods, there are Masuko Sanyo's super masscolloider grinder method, Microfluidics' Microfluidizer method, Silverson's high pressure homogenizer method and the like.

Among these mechanical methods for processing cellulose, the grinder method produces cellulose nanofibrils by passing cellulose between a pair of disks whose upper and lower portions are coupled to each other. The high pressure homogenizer method produces cellulose nanofibrils by passing cellulose through a thin and bent tube under very high pressure. Therefore, it has been known that properties of the prepared cellulose nanofibrils are uniformly determined due to characteristics of each of these devices. In addition, with respect to the properties of the cellulose nanofibrils to be prepared, it has become a major issue to produce a product having a long length and a thin thickness.

Among the prior arts, Korean Patent Registration Publication No. 10-1487475 proposes a method for fabricating nano cellulose fiber including the steps of: preparing a suspension containing cellulose fibers, performing a complex process for pulverizing, homogenizing and crushing the cellulose fibers in the suspension, and dehydrating the suspension to obtain nano cellulose fibers in a solid powder form, and dispersing the nano cellulose fibers in the agglomerated solid powder form.

In addition, Korean Patent Laid-Open Publication No. 10-2015-0110549 discloses cellulose nanofiber in which a portion of a hydroxyl group is substituted with a carboxyl group and an aldehyde group, wherein fine cellulose fibers have a maximum fiber diameter of 1,000 nm or less, and a number average fiber diameter of 2 nm or more and 150 nm or less.

It is confirmed that the modified cellulose prepared as described above has a chemical structure or some properties similar to that of hyaluronic acid, which is widely used in conventional cosmetic compositions.

Formula 1 below represents a chemical structure of carboxymethyl cellulose (CMC), which is a type of typical modified cellulose, and Formula 2 below represents a chemical structure of hyaluronic acid.

[Formula 1]

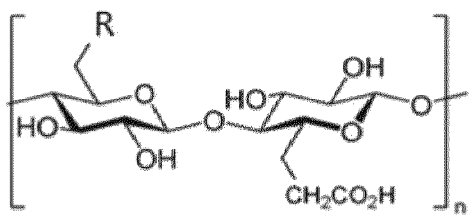

R=OH (=CNF) or R=$CH_2CO_2H$

[Formula 2]

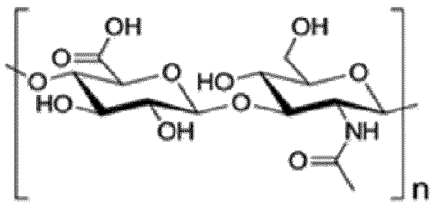

As described above, the modified cellulose has a chemical structure similar to that of hyaluronic acid with excellent biocompatibility, but has physical properties different therefrom, thereby making it difficult to use as a replacement raw material for the hyaluronic acid.

However, some patents suggest a dermal filler in which hyaluronic acid and carboxymethyl cellulose are mixed. As an example, Korean Patent Publication No. 10-2017-0117368 propose an injectable dermal filler composition which includes cross-linked hyaluronic acid (HA), carboxymethyl cellulose (CMC), and optionally microparticles such as calcium hydroxyapatite (CaHAP) microparticles.

Further, US Patent Registration Publication No. 9371402 proposes a dermal filler configuration containing hyaluronic acid, carboxymethyl cellulose, and a crosslinking agent.

In addition, International Publication No. WO 2007-014285 proposes a composition including carboxymethyl cellulose (CMC), and polyethylene oxide (PEO), and the use thereof as a filler for injecting the composition into the skin at sites where filling in the skin is desired.

As described above, there has been proposed the cases in which carboxymethyl cellulose, which is a modified cellulose, is mixed with various substances and used as a dermal filler.

However, such carboxymethyl cellulose has not yet been proposed as a single ingredient for use as a dermal filler, and is generally used as a just a minimal part of supplementary component depending on a bio-derived natural raw material such as hyaluronic acid.

In particular, dermal fillers made of natural raw materials such as hyaluronic acid have very limited application ingredients, and it is difficult to be widely used due to a high price thereof.

Further, since the existing filler has a short cycle of biodegradability, it is inconvenient to reuse the filler at a short cycle of 6 months or 2 to 3 years depending on the raw material component thereof.

[Prior Art Document]

[Patent Document]

(Patent Document 001) Korean Patent Registration Publication No. 10-1487475

(Patent Document 002) Korean Patent Publication No. 10-2015-0110549
(Patent Document 003) Korean Patent Publication No. 10-2017-0117368
(Patent Document 004) US Patent Registration Publication No. 9,371,402
(Patent Document 005) International Patent Publication No. WO 2007-014285

[Summary of Invention]

[Problems to be Solved by Invention]

In order to solve the above-described problems of the prior art, a problem to be solved by the present invention is to provide a composition to be used as a skin beauty enhancing composition such as a filler for tissue repair, by preparing modified cellulose among ingredients that have been used as fillers in the art under specific conditions and configuring so as to have specific physical properties.

Accordingly, an object of the present invention is to provide a dermal filler composition including modified cellulose in a cellulose nanofibril (CNF) form having specific properties of thin and short in thickness and length as an effective ingredient.

In addition, another object of the present invention is to provide a dermal filler composition containing carboxymethyl cellulose (CMC), which is a modified cellulose of a novel configuration having new physical properties under specific conditions.

Further, another object of the present invention is to provide a skin beauty enhancing composition containing

carboxymethyl cellulose having specific physical properties.

[Means for Solving Problems]

To solve the above-described objects, according to an aspect of the present invention, there is provided a dermal filler composition including modified cellulose in a cellulose nanofibril form as an effective ingredient, wherein the modified cellulose is made of pulp cellulose, contains a carboxymethyl group as a substituent, and has an average diameter of 3.0 to 10.0 nm and a length of 100 to 560 nm.

According to a preferred embodiment of the present invention, there is provided a dermal filler composition including modified cellulose which contains a substituent substituted with a carboxymethyl group and has a water retention value (WRV) of 200 to 450%, which is obtained by measuring a water content after centrifugation for 50 minutes under 968G (gravitational acceleration) conditions.

In addition, according to another aspect of the present invention, there is provided a skin beauty enhancing composition including modified cellulose which has a viscosity of 3,000 to 10,000 cps.

In addition, according to a preferred embodiment of the present invention, there is provided a dermal filler composition including modified cellulose which has a tan $\delta$ value of a loss modulus to a storage modulus in a range of 0.1 to 0.3 under a low shear stress condition of 0.1 Pa, and a tan $\delta$ value of the loss modulus to the storage modulus in a range of 1.0 to 2.0 under a high shear stress condition of 25 Pa.

Further, according to a preferred embodiment of the present invention, preferably, the dermal filler composition of the present invention has a storage modulus of 600 to 1,200 Pa, and a loss modulus of 150 to 400 Pa.

Further, according to a preferred embodiment of the present invention, there is provided a dermal filler composition which has a substitution degree of 0.3 to 0.6.

Further, according to another aspect of the present invention, there is provided a facial filler including the dermal filler composition.

Furthermore, according to another aspect of the present invention, there is provided a skin beauty enhancing composition having the above-described physical properties.

[Advantageous Effects]

The modified cellulose according to the present invention has water retention value within a specific range while having excellent moisturizing power, viscosity, storage modulus, loss modulus, etc., and has good biodegradability without cytotoxicity. Therefore, it was confirmed that the modified cellulose has an excellent skin tissue repair effect.

In particular, when injecting the filler into the skin, since the modified cellulose has an excellent skin tissue repair effect with properties similar to hyaluronic acid, it may be widely used as a replacement material for expensive hyaluronic acid.

Therefore, the dermal filler composition containing the modified cellulose of the present invention may be applied to the facial filler and the like.

In addition, since the modified cellulose having specific physical properties of the present invention has a similar to or better skin-beautifying effect than hyaluronic acid, it may be replaced with the skin beauty enhancing composition to which expensive hyaluronic acid is currently applied, and may be used very economically.

[Brief Description of Drawings]

FIG. 1 is a flowchart illustrating a method for manufacturing modified cellulose according to the present invention.

FIG. 2 is a graph for comparing physical properties of cellulose nanofibrils which vary depending on the number of times that pulp cellulose pretreated according to the present invention is input into the grinder in preparative examples of the present invention.

FIG. 3 is a graph for comparing physical properties of modified cellulose nanofibrils which vary depending on the number of times of being input when fabricating by inputting the pulp celluloses pretreated according to the present invention in the comparative examples and examples of the present invention or the modified celluloses in a nanofibril form prepared through a grinder treatment step into a high pressure homogenizer.

FIG. 4 is a graph illustrating moisturizing power evaluation results by comparing according to the results in which pretreatment of pulp cellulose, grinder treatment after pretreatment, and all the pretreatment, grinder treatment, and high pressure homogenizer treatment are performed according to the method for preparing the modified cellulose in the nanofibril form in experimental examples.

FIG. 5 is a graph illustrating experimental results of water retention value by comparing the modified cellulose in

the nanofibril form according to the present invention.

FIGS. 6A and 6B are graphs illustrating tan δ values of a loss modulus to a storage modulus of the modified cellulose in the nanofibril form according to the present invention under a low shear stress condition of 0.1 Pa (FIG. 6A) and a high shear stress condition of 25 Pa (FIG. 6B) by comparing with hyaluronic acid, respectively.

FIGS. 7A and 7B are graphs illustrating cytotoxicity test results for kidney cells (FIG. 7A) and hepatocytes (FIG. 7B) of the modified cellulose in the nanofibril form according to the present invention by comparing with hyaluronic acid, respectively.

[Mode for Carrying out Invention]

Hereinafter, the present invention will be described in more detail as one embodiment as follows.

The present invention relates to modified cellulose having new physical properties and novel use of such modified cellulose. In particular, the present invention relates to a new concept of modified cellulose which may have an excellent moisturizing effect as well as skin tissue repairability thus to be useful in a skin beauty enhancing composition such as a dermal filler, by preparing cellulose under specific conditions, and has excellent physical properties such as hygroscopic property, water retention value, viscosity, storage modulus and loss modulus.

In the present invention, cellulose obtained from wood-based or non-wood-based pulp may be used. The term "wood-based pulp" includes all kinds of wood, pulp obtained from plants, fruits, natural resources, etc. having the same material as wood. The term "non-wooden-based pulp" refers to agricultural by-products such as rice straw, wheat straw, corn stalk, sorghum stalk, sugar cane by-products, cottonseed seed coat fiber, bamboo and the like. For example, cotton cellulose, which is a seed coat fiber surrounding the cottonseed, may be used as the non-wood-based cellulose.

Preferably, the modified cellulose of the present invention may be made of cellulose derived from the wood-based pulp. Most preferably, the case of using bamboo pulp cellulose is usefully applied. In addition, as the non-wood-based pulp, cottonseed seed coat fiber cellulose may be preferably used.

According to a preferred embodiment of the present invention, pulp cellulose which is subjected to an oxidation or substitution pretreatment process is preferably applied as the cellulose. Such pretreated pulp cellulose may be, for example, cellulose containing one or more of carboxymethylated, aminated or TEMPO-oxidized substituents.

In the present invention, the modified cellulose is preferably modified cellulose in which a carboxymethyl group is substituted.

As used herein, the term "substituted" means that an OH group at an end of cellulose is substituted with a carboxymethyl group or the like.

As used herein, the "substitution degree" indicates a degree in which the OH group of an end group is substituted with a substituent such as a carboxymethyl group in the chemical structure of the cellulose. When a substitutable group is not substituted at all, the substitution degree is 0, and when OH groups of all substitutable end groups are substituted, the substitution degree is 1. Therefore, the substitution degree may be expressed by being greater than 0 and limiting the maximum value to 1 depending on the substituted degree.

As used herein, the term "water retention value (WRV)" refers to a numerical value expressed as a percentage of a result value obtained by measuring a water content after centrifugation of the modified cellulose for 50 minutes under 968G (gravitational acceleration) conditions.

As used herein, the term "viscosity" refers to a viscosity measured by diluting the modified cellulose at a concentration of 0.5 to 5 % by weight ('wt.%').

As used herein, the "storage modulus" is an index indicating a degree of sagging due to gravity after skin injection, and refers to a storage coefficient measured under a low shear stress condition of 0.1 Pa, which is a condition after skin injection.

In addition, as used herein, the "loss modulus" is an index indicating a degree of ease of injection, which is the property that a liquid tends to flow during injection before skin injection, and refers to a loss coefficient measured under a high shear stress condition of 25 Pa, which is a condition before skin injection.

Herein, the storage modulus is a coefficient related to the property of a solid or gel to resist the flow of a liquid, and the loss modulus is a coefficient related to the property of the liquid to flow.

According to a preferred embodiment of the present invention, the dermal filler composition or skin beauty enhancing composition of the present invention includes modified cellulose as an effective ingredient, and particularly pulp cellulose. It is preferable that the inventive composition includes modified cellulose which contains a carboxymethyl group as a substituent, and has an average diameter of 3.0 to 10.0 nm, and a length of 100 to 560 nm.

According to a preferred embodiment of the present invention, among the modified celluloses, cellulose nanofibril (CNF) may be made of cellulose essentially having properties of a thin thickness and a short length.

According to a preferred embodiment of the present invention, the modified cellulose of the present invention has a nanofibril form. More preferably, the modified cellulose has an average diameter of 3.5 to 5.0 nm in fibers.

According to a preferred embodiment of the present invention, more preferably, the modified cellulose has a

length of 120 to 500 nm.

According to a preferred embodiment of the present invention, more preferably the modified cellulose has an average length of 300 nm or more.

According to the present invention, the composition containing such modified cellulose may be typically used in a hydrogel form. According to a preferred embodiment of the present invention, the hydrogel contains modified cellulose capable of being mixed in water, in a content of 0.1 to 10 wt.%, and preferably 0.5 to 5 wt.% based on a solid content. If the solid content is too low, it is difficult to use the hydrogel as a dermal filler composition or skin beauty enhancing composition because it is too thin, and if the content thereof is too high, the viscosity is also high such that it is difficult to use the hydrogel as the inventive composition.

According to a preferred embodiment of the present invention, when the modified cellulose does not have the average diameter and length in fibers within the above range, a moisturizing power is significantly reduced. In addition, if the average length is too small, other physical properties may be greatly reduced.

According to a preferred embodiment of the present invention, the dermal filler composition of the present invention contains a substituent substituted with a carboxymethyl group. The inventive composition preferably includes modified cellulose having a water retention value (WRV) of 200 to 450%, and preferably 280 to 400%, which is obtained by measuring a water content after centrifugation for 50 minutes under 968G (gravitational acceleration) conditions.

Further, according to a preferred embodiment of the present invention, the dermal filler composition of the present invention has a viscosity of 3,000 to 10,000 cps, and preferably 5,000 to 8,000 cps.

In addition, according to a preferred embodiment of the present invention, there is provided a dermal filler composition including modified cellulose, which has a tan $\delta$ value (loss modulus/storage modulus) of a loss modulus to a storage modulus in a range of 0.1 to 0.3, and preferably 0.12 to 0.20 under a low shear stress condition of 0.1 Pa, which is a skin injection condition, and a tan $\delta$ value of the loss modulus to the storage modulus in a range of 1.0 to 2.0, and preferably 1.05 to 1.50 under a high shear stress condition of 25 Pa. Herein, the tan $\delta$ value is close to 0 under the low shear stress condition, such that long-term retention of the filler is excellent compared to that of hyaluronic acid. In addition, the tan $\delta$ value is greater than 1 under the high shear stress condition, such that the modified cellulose is in a liquid state before injection into the skin, and thereby having excellent physical properties that facilitate injection.

In addition, according to a preferred embodiment of the present invention, the dermal filler composition of the present invention has a storage modulus of 600 to 1,200 Pa, and preferably 700 to 1,100 Pa, and a loss modulus of 150 to 400 Pa, and preferably 200 to 350 Pa.

The composition according to the present invention has similar to or better physical properties than hyaluronic acid without cytotoxicity, and has excellent storage modulus and loss modulus at a low shear stress, which is the skin injection condition, and thereby having physical properties suitable as a filler.

In addition, since the modified cellulose according to the present invention has physical properties such as water retention value, tan $\delta$ values at low shear stress and high shear stress, storage modulus, and loss modulus as described above, it may be replaced with the skin beauty enhancing composition to which expensive hyaluronic acid is currently applied, and may be widely used.

Accordingly, the dermal filler composition of the present invention having the above-described physical properties includes the use as a skin beauty enhancing composition. Specifically, the present invention includes a cosmetic composition having functions such as moisturizing and wrinkle improvement.

In particular, according to a preferred embodiment of the present invention, it is preferable that the dermal filler composition of the present invention includes modified cellulose having a substitution degree of 0.3 to 0.6.

According to a preferred embodiment of the present invention, it is preferable that the dermal filler composition or skin beauty enhancing composition of the present invention simultaneously satisfy two or more of various physical property conditions such as the above-described water retention value, tan $\delta$ values at low shear stress and high shear stress, storage modulus, loss modulus, viscosity, substitution degree and the like. It is more preferable that the inventive compositions satisfy all of the above-described physical property conditions.

According to a preferred embodiment of the present invention, the modified cellulose of the present invention as described above may be manufactured through a novel combination of mechanical treatment processes.

In particular, according to the novel manufacturing method of the present invention, by nanofibrillating the modified cellulose to be prepared, it is possible to control the average diameter and length thereof.

Therefore, when using the manufacturing method of the present invention, unlike conventional cellulose, in particular, conventional carboxymethylated cellulose (CMC), cellulose nanofibrils having a thin thickness, short length, and excellent moisturizing power may be manufactured in large quantities and may be economically manufactured.

In addition, according to a preferred embodiment of the present invention, by controlling the substitution degree in the carboxymethyl group substitution process, which is a pretreatment process performed at the beginning of the manufacturing process, and controlling the manufacturing process conditions, modified cellulose satisfying the above-described various properties may be manufactured.

According to a preferred embodiment of the present invention, in order to prepare elongated modified cellulose

nanofibrils (CNFs) according to the present invention, treatment steps as shown in the process diagram of FIG. 1 as one embodiment may be performed.

Hereinafter, a method for manufacturing modified cellulose according to the present invention will be described as one embodiment.

According to a preferred embodiment of the present invention, in order to produce long and thin nanofibrillated modified cellulose, it is preferable that the modified cellulose is subjected to all of a grinder treatment step for basically grinding cellulose and a high pressure homogenizer treatment step for performing highpressure homogenization.

According to a preferred embodiment of the present invention, as the raw material for pulp used in the present invention, both wood-based and non-wood-based materials may be used. That is, all pulp may be used as the cellulose nanofibril material, and in particular, even in the case of bamboo pulp having a rough surface, it has been demonstrated to be very effective when using as a nano-sized material.

According to a preferred embodiment of the present invention, in order to prepare elongated modified cellulose, pulp cellulose is first subjected to a pretreatment step for facilitating preparation of nano-sized fibers.

Herein, the pretreatment is a technique of pretreating cellulose of a pulp phase before inputting it into a main process so as to have specific properties, in order to more efficiently prepare nanofibrils which are modified celluloses of specific properties. For example, the pretreatment may be performed through carboxymethylation of attaching a carboxymethyl group to cellulose, or catalytic oxidation using a TEMPO derivative.

Carboxymethylation applicable in the present invention is a method of substituting $CH_2$-COOH on a surface of cellulose. That is, this technique allows the functional group to be substituted on the surface of the cellulose, such that the surface has a negative charge, and thereby causing an electrostatic repulsive force to be acted thereon due to the negative charge between the celluloses.

In addition, the catalytic oxidation using a TEMPO derivative is a treatment for imparting an electrostatic repulsive force by oxidizing number 6 carbon ($CH_2OH$) of cellulose to COOH, such that the surface of the cellulose has a negative charge.

According to a preferred embodiment of the present invention, for example, when performing carboxymethylation in the pretreatment process, the negative charge on the surface of the cellulose acts to cause a repulsive force to act between the cellulose fibers by an electrostatic repulsive force. Therefore, manufacturing productivity of cellulose nanofibrils may be greatly increased.

According to a preferred embodiment of the present invention, the pulp cellulose pretreatment step is a step of preparing a pulp material before inputting it into a main production process by mixing a chemical substance with the pulp cellulose and stirring at a specific temperature for a predetermined period of time. Through this step, it is possible to greatly increase production efficiency of nanofibrils in the main production process.

In the present invention, the pretreatment may be performed using a slight amount of pulp cellulose, sodium hydroxide, and monochloroacetic acid (liquid phase). In this case, the respective components are mixed with each other, for example, in amounts of 1 to 10 parts by weight of pulp cellulose, 0.1 to 3 parts by weight of sodium hydroxide, and 0.1 to 2 parts by weight of monochloroacetic acid to prepare a liquid mixture, followed by treating the mixture at 85°C to 95°C for 100 minutes to 140 minutes while stirring the same.

According to a preferred embodiment of the present invention, the method comprises a grinder treatment step for converting the pretreated pulp cellulose into cellulose nanofibrils through a grinder device.

Herein, for example, it is preferable to add purified water to the pretreated pulp cellulose to prepare a suspension so that the pulp cellulose content is 0.5 to 3 wt.%. If the pulp cellulose content of the suspension input to the grinder is less than 0.5%, a frictional force between disks in contact with the cellulose is largely decreased, and the production efficiency is greatly reduced. If the content thereof exceeds 3%, the viscosity is greatly increased and workability is significantly reduced. For this reason, it is not preferable in terms of production costs.

In the grinder method, upper and lower portions of the disks are coupled to rotate in directions opposite to each other, and cellulose is input into a minute gap formed between the disks during operation. At this time, it is known that the properties of the cellulose nanofibrils may be variously controlled by controlling the number of times of cellulose input and a gap through which the cellulose passes, which is set in the device. In addition, the high pressure homogenizer is equipped with an 'L'-shaped thin tube therein, and a cellulose suspension passes through the tube to make the suspension mechanically collide with an angled portion of the tube, which is a technique to make nano-sized cellulose.

In this case, as a grinder for preparing nano cellulose to be used, a Colloidal Mill from IKA Com., or a Supermasscolloider from Masuko Sangyo may be commonly used.

The grinder is operated in such a manner that a pair of disks are coupled and rub against each other. At this time, by controlling the gap between the disks through which the cellulose passes, cellulose having a nano size is prepared. That is, by controlling the state of the cellulose to be input and the gap between the disks of the grinder, nano-sized cellulose having various properties is prepared. Up to now it is known that the cellulose nanofibrils to be used in industry should be subjected to treatment about 15 to 30 times.

The nanofibril strands of modified cellulose prepared through the grinder treatment step have specific properties.

According to a preferred embodiment of the present invention, the method may include the high pressure homogenizer treatment step of retreating the nanofibrils of the modified cellulose that has been subjected the grinder treatment step using a device such as a high pressure homogenizer.

According to a preferred embodiment of the present invention, the nanofibrils of the modified cellulose prepared through the grinder treatment step are subjected to the high pressure homogenizer treatment step of inputting them into a high pressure homogenizer for more detailed processing. Then, nanofibrillated modified cellulose may be prepared in a size that can be used for specific applications.

That is, according to the present invention, preferably, the modified cellulose that has been subjected to the grinder treatment step is further subjected to the high pressure homogenizer treatment step, such that nanofibrils of modified cellulose having specific properties due to their unique thickness and length may be mechanically prepared in large quantities, which are completely different from the cellulose nanofibrils prepared only by the grinder or only by the high pressure homogenizer.

According to a preferred embodiment of the present invention, it is desired that the grinder treatment step and the high pressure homogenizer treatment step are preferably repeated 1 to 10 times, more preferably 2 to 6 times, and most preferably 4 times.

The modified cellulose prepared as described above has a significantly increased specific surface area than nanofibrils prepared by the conventional method. Therefore, it is expected that modified cellulose may be widely used in specific industrial fields such as cosmetics that require high water retention and moisturizing power.

As described above, the grinder treatment or the high pressure homogenizer treatment methods, which are techniques applied in the present invention, are only each independently introduced and utilized at the nano-modified cellulose manufacturing site, and there is no example of preparing modified cellulose having preferable physical properties as in the present invention in combination of these two methods.

The modified cellulose of the present invention may be easily manufactured by using a specific combination of the grinder method and the high pressure homogenizer method. In particular, nanofibrils of modified cellulose having a thin thickness and a short length may be prepared in large quantities.

Preferably, the present invention adds the process of pretreating the pulp cellulose before inputting it into the full-scale manufacturing process, thus to further increase the production efficiency in the main process. Thereby, it is possible to prepare a large amount of modified cellulose having physical properties required by the present invention.

As described above, the nanofibrils of the modified cellulose prepared according to the present invention have a thinner thickness or a shorter length than the conventional products. After preparing a 1% suspension of cellulose nanofibrils made of nano cellulose, by maintaining a residual weight of 75% or more after 4 hours, for example 75 to 85%, 65% or more after 6 hours, 50% or more after 8 hours, for example 50 to 60%, and 22% or more after 12 hours, for example 22 to 35% under a temperature of 25°C and a relative humidity of 50%, such that it is possible to exhibit more superior moisturizing power than the conventional products.

In particular, the modified cellulose in the cellulose nanofibril form according to the present invention may maintain water retention value, tan $\delta$ values at low shear stress and high shear stress, storage modulus, loss modulus, viscosity, substitution degree, and the like under the above-described conditions.

Therefore, the modified cellulose of the present invention as described above may be preferably used as the dermal filler composition or the skin beauty enhancing composition.

The composition according to the present invention may be used as a material in the cosmetic field when strong moisturizing power is required, and may be used as a substitute for hyaluronic acid. Further, in addition to cosmetics, processed foods for diet, disposable diapers, electrical and electronic materials, biomedical materials, or nanocomposite materials, and the like may be processed utilizing the composition according to the present invention.

Hereinafter, the present invention will be described in detail based on examples, but the present invention is not limited to the examples.

1. Pulp cellulose pretreatment step

For pretreatment, 3 to 6 parts by weight of pulp cellulose, 0.6 to 1.5 parts by weight of sodium hydroxide, and 0.4 to 0.6 parts by weight of monochloroacetic acid were mixed, and the mixture was added to 92 to 96 parts by weight of ethanol, followed by thorough stirring. The pulp cellulose liquid mixture made through the above process was stirred at a temperature of 80°C to 95°C for 100 minutes to 140 minutes.

Herein, it is most preferable that the composition of the liquid pulp cellulose mixture includes 4.7 parts by weight of kraft pulp, 0.9 parts by weight of sodium hydroxide, 0.5 parts by weight of monochloroacetic acid, and 93.9 parts by weight of ethanol in this treatment step. Under the conditions of temperature and reaction time, if the temperature is too low, the reaction time is set to be rather long, and if the temperature is rather high, it is preferable to control the reaction time to be relatively short. The reason is that at a temperature lower than 80°C, a reaction speed of cellulose with the components in the liquid product is greatly reduced, thereby resulting in a reduction of efficiency, and at a high temperature

exceeding 95°C, an explosion risk of ethanol, the main component, is greatly increased, which is an unfavorable condition at the production site. The most preferred reaction temperature and time is 120 minutes at 90°C, respectively.

[Preparative Example 1]

100 g of kraft pulp (manufactured by Moorim PNP, bleached hardwood pulp), 20 g of sodium hydroxide, and 10 g of monochloroacetic acid were mixed in 2 kg of liquid ethanol to prepare a pulp liquid product, then the mixture was stirred at 90°C for 120 minutes to pretreat pulp cellulose.

[Preparative Example 2]

Except for controlling monochloroacetic acid to 6 g, other components were used in the same manner as in Preparative Example 1 to prepare pulp cellulose.

[Preparative Example 3]

Except for controlling monochloroacetic acid to 14 g, other components were used in the same manner as in Preparative Example 1 to prepare pulp cellulose.

[Preparative Experimental Example 1]

As a result of the experiment on the celluloses prepared in Preparative Examples 1 to 3, it was confirmed that the pulp cellulose of Preparative Example 3 exhibited an excessive chemical reaction of monochloroacetic acid to turn transparent, and a dehydration property was significantly decreased.

From this result, it was confirmed that a composition ratio of monochloroacetic acid in the pretreatment step of the present invention was preferably controlled to less than 0.7 parts by weight.

2. Grinder treatment step

The cellulose that has been subjected to the above '1. Pulp cellulose pretreatment step' was mechanically treated using a grinder to prepare cellulose nanofibrils.

0.5 to 3 parts by weight of pulp cellulose prepared in a slurry state through Preparative Example 1, and 97 to 99.5 parts by weight of purified water were mixed to prepare a 0.5 to 3% suspension, followed by passing through a grinder equipment while stirring to be sufficiently dispersed using a homogenizer.

If a pulp cellulose content of the suspension injected into the grinder is less than 0.5%, a frictional force between disks in contact with the cellulose is largely decreased and production efficiency is greatly reduced. If the content thereof exceeds 3%, the viscosity is greatly increased and workability is significantly reduced. For this reason, it is not preferable in terms of production costs.

Since a gap formed between the disks of the grinder is an important factor that provides a shear force by applying friction to the input cellulose, an experiment was performed by setting conditions for manufacturing customized cellulose nanofibrils as follows.

[Preparative Example 4]

100 g of the pretreated cellulose prepared in Preparative Example 1 was added to 5 kg of purified water to prepare a suspension using a homogenizer, and then added to a Supermasscolloider grinder from Masuko Sangyo. At this time, a rotation speed of the grinder was set to 1,300 rpm, a gap formed between the disks during input was controlled to 100 to 150 μm, and the number of times of input was set to 3 times.

[Preparative Example 5]

The same procedures as described in Preparative Example 4 were performed, except that the number of times of input was controlled to one time.

[Preparative Example 6]

The same procedures as described in Preparative Example 4 were performed, except that the number of times of input was controlled to 6 times.

A viscosity (cp) of each of the cellulose nanofibrils prepared in Preparative Examples 4 to 6 was measured, and the measurement method and results are as follows. Viscosity measurement was performed with a Brookfield viscometer, and the measurement was performed by diluting the nano cellulose at a concentration of 1%. Average thickness and length of the prepared cellulose nanofibrils were measured using TEM (manufactured by Carl Zeiss, trade name Libra 120), and measured results are shown in Table 1 below.

[TABLE 1]

| | Thickness of nanofibril (average) | Length of nanofibril (range, average) | Viscosity (cp) |
|---|---|---|---|
| Preparati ve Example 4 | 11 nm | 132 nm - 497 nm(366 nm) | 787.4 |
| Preparati ve Example 5 | 25 nm | 172 nm - 1,660 nm(791 nm) | 671.2 |
| Preparati ve Example 6 | 10 nm | 128 nm - 429 nm(276 nm) | 825.7 |

From Table 1 and FIG. 2, when the gap formed between the disks of the grinder was 90 μm, physical properties of the pulp cellulose after treatment were compared. It was confirmed that when the number of times of input was 2 times, the thickness was significantly increased compared to the case of inputting one time, but compared with the case of inputting 5 times, the thickness and length of the fibril were quite similar except for the viscosity. From this result, in relation to the number of times of grinder treatment for preparing cellulose nanofibrils having a thin thickness and a short length, it was confirmed that the most effective treatment was 3 times when the gap between the disks was 100 to 150 μm. This exhibits a very meaningful technical characteristic in relation to the pretreatment step in controlling the thickness and length, compared to the conventional method of performing treatment about 15 to 30 times.

3. High pressure homogenizer treatment step

100 g of the pretreated cellulose prepared in the above '1. Pulp cellulose pretreatment step' and '2. Grinder treatment step' was input to 5 kg of purified water to prepare a suspension using a homogenizer, and then input it into a high pressure homogenizer to prepare cellulose nanofibrils.

Since the cellulose nanofibrils prepared through this process are input in a slurry state containing purified water, workability is greatly reduced by frictional force. Therefore, the physical properties of the final product were confirmed while properly controlling the pressures at the time of initial input and at the time of re-input.

[Comparative example]

100 g of the pulp cellulose pretreated in Preparative Example 1 was input into a high pressure homogenizer under a pressure of 100 bar at the time of initial input, and then treated repeatedly 5 times under a pressure of 800 bar.

[Example 1]

100 g of the cellulose nanofibrils prepared in Preparative Example 4 were put into a high pressure homogenizer 3 times under a pressure of 100 bar at the time of initial input, and then treated repeatedly 2 times under a pressure of 800 bar.

[Example 2]

100 g of the cellulose nanofibrils prepared in Preparative Example 5 were put into a high pressure homogenizer 3 times under a pressure of 100 bar at the time of initial input, and then treated repeatedly 2 times under a pressure of 800 bar.

[Example 3]

100 g of the cellulose nanofibrils prepared in Preparative Example 6 were put into a high pressure homogenizer 3 times under a pressure of 100 bar at the time of initial input, and then treated repeatedly 2 times under a pressure of 800 bar.

[Experimental Example 1]

Average thickness and length of the nanofibrillated modified cellulose prepared in the comparative example and Examples 1 to 3 were measured using TEM (manufactured by Carl Zeiss, trade name Libra 120), and measured results are shown in Table 2 below and a graph for comparison in FIG. 2.

[TABLE 2]

| Number of times of input | Thickness of nanofibril (average) | Length of nanofibril (range, average) |
|---|---|---|
| Comparative example | 9.0 nm | 349 nm - 1,513 nm (726 nm) |
| Example 1 | 4.3 nm | 125 nm - 492 nm(316 nm) |
| Example 2 | 6.6 nm | 145 nm - 718 nm(429 nm) |
| Example 3 | 3.7 nm | 123 nm - 421 nm(213 nm) |

From Table 2 and FIG. 3, as in Example 1, when treating 3 times in a grinder and 3 times in a high pressure homogenizer, the highest quality of cellulose nanofibrils was obtained compared to the pretreated pulp cellulose subjected to the treatment 6 times.

In particular, it was confirmed that, as the thickness and length of the fibrils were decreased, the nanofibrils of Example 3 exhibited a significant improvement effect in terms of viscosity (cP), which is a criterion for evaluating the moisturizing power, compared to Example 1. That is, it was confirmed that the nanofibrils of Example 1 exhibited effects of improving about 46.3% in the thickness and about 40% in the length, and an excellent improvement effect of about 13% in terms of viscosity for evaluating the moisturizing power compared to the comparative example.

However, as a result of comparing Example 1 and Example 3, even when treating 6 times in the grinder and the high pressure homogenizer, the improvement effect was not significant compared to the case of treating 3 times in the grinder and the high pressure homogenizer, respectively, and also exhibited very similar results in terms of the viscosity. From this result, it was confirmed that, after the carboxylation step, the case of respectively treating 3 times in the grinder and the high pressure homogenizer exhibited the most excellent physical properties in terms of cost-effectiveness.

[Experimental Example 2] Moisturizing power evaluation experiment

A moisturizing power among the physical properties of the cellulose nanofibrils obtained in the comparative example and Examples 1, 2 and 3 was evaluated, which were prepared by parallel treatment of the grinder method and the high pressure homogenizer method.

The moisturizing power could be confirmed by leaving the product at room temperature and measuring the degree of moisturizing as time elapsed. For the experiment, 10 g of cellulose nanofibrils prepared in the comparative example and Examples 1, 2 and 3, respectively, were put in a beaker on which a filter paper was placed under a temperature of 25°C and a relative humidity of 60%, and evaporation amounts for 6 hours, 12 hours, 18 hours and 24 hours were measured to determine the remaining weight (wt.%) at each predetermined time. Measured results are shown in Table 3 below and a graph for comparison in FIG. 4.

[TABLE 3]

| Number of times of input | After 6 hours | After 12 hours | After 18 hours | After 24 hours |
|---|---|---|---|---|
| Comparative example | 69.1 | 28.2 | 11.2 | 7.2 |
| Example 1 | 70.5 | 40.1 | 15.8 | 12.9 |
| Example 2 | 67.8 | 22.3 | 12.0 | 5.7 |
| Example 3 | 69.1 | 22.2 | 5.9 | 3.1 |

As can be seen from Table 3, in the nanofibrils of Example 1, the size of the nano cellulose was reduced after the grinder process and the high pressure homogenizer process were conducted, and hydroxyl groups of the nano cellulose were exposed, such that the moisturizing power was significantly increased compared to the comparative example and Example 2. However, in the case of the nanofibrils of Example 3, in which the size of the nano cellulose was further decreased, the amount of exposed hydroxyl groups was increased, but the amount of moisture per fiber

was decreased, such that the moisturizing power was reduced.

As can be seen from Table 3 above and FIG. 4, it could be confirmed that the pulp cellulose which has been subjected to carboxymethylation had significantly improved moisturizing power when respectively treating 3 times by the grinder and the high pressure homogenizer, compared to the nanofibrils respectively treated 6 times by the grinder or the high pressure homogenizer. It has been proved that, as can be seen from the measured thickness and length of the nanofibrils, the moisturizing power was greatly increased as the nanofibrils are further thinned and shortened.

[Experimental Example 3] Evaluation of water retention value (WRV)

When CNFs were used as a facial filler, water retention was measured to test a degree of water retention value in the human body.

1 g of kraft pulp was homogenized by a homogenizer (IKA) at 6000 to 8000 rpm. The homogenized pulp was filtered by a glass filter. This process is to prevent CNFs from escaping by laying a pulp mesh on the glass filter.

Next, the pulp mesh was compressed by centrifugation under 968G (gravitational acceleration) conditions at 25°C for 15 minutes using an H-103NR (model name) centrifuge from Kokusan Enshinki Co., Ltd.

About 8 g of a sample to be measured was put in the same centrifuge, and centrifuged under 968 G (gravitational acceleration) conditions for 50 minutes. After centrifugation, weights of the CNFs and pulp mesh before and after drying were measured.

The pretreated CNF samples for each substitution degree were diluted to 1 wt.%, followed by treating using a grinder (Supermasscolloider, Masuko Sangyo) 4 times and a high pressure homogenizer (Panda plus, GEA) 8 times. Based on the measured results, the water retention value (WRV) was calculated by a method shown in Equation 1 below.

[Equation 1]

$$\text{WRV}(\%) = \{(\text{Weight of sample - Weigh of sample after drying})/\text{Weigh of sample after drying}\} \times 100 - \text{Weigh of pulp mesh}$$

The WRV was measured by the above method, but the WRV value measured for each substitution degree of the carboxymethyl group with respect to CNF was compared with the value of hyaluronic acid (HA) under the same conditions, and results thereof are shown in Table 4 below.

[TABLE 4]

| Substitutio n degree of CNF | 0.2 | 0.3 | 0.5 | 0.6 | 0.7 | HA |
|---|---|---|---|---|---|---|
| WRV (%) | 55 | 303 | 378 | 326 | 69 | 229 |

These results are the same as those shown in the graph of FIG. 5. According to these results, it was confirmed that an intrinsic water retention value (WRV) of Kraft pulp in the case of substitution degree of 0.3 to 0.6 was significantly superior to other cases.

From the above experimental results, it was confirmed that, in the case of CNFs, the case of substitution degree of 0.3 to 0.6 had WRV of 303 to 326, and retained moisture about threefold (300%) or more, which is superior to hyaluronic acid.

[Experimental Example 4] Evaluation of rheology modifier

In a case of using the CNFs as a facial filler, in order to determine a rheology modifier (i.e., a change in fluidity) when fixed in the human body and when injected into the human body, a storage modulus and a loss modulus when applying a low shear stress and a high shear stress were determined, respectively.

Measurement was performed using a rheometer (MCR102, Anton Paar, Austria) as a device for measuring the rheology modifier, and a measuring plate PP25 was used as a measuring system of a measuring tool. The measurement was performed in a linear viscoelastic range mode as a measurement mode, and the storage modulus and the loss modulus were measured at stresses from 0.1 Pa (low shear stress) to 25 Pa (high shear stress).

The pretreated CNF samples for each substitution degree were diluted to 1 wt.%, followed by treating using a grinder (Supermasscolloider, Masuko Sangyo) 4 times and a high pressure homogenizer (Panda plus, GEA) 8 times.

The low shear stress condition (0.1 Pa) assumes a state in which the facial filler is injected into the skin and

then fixed. In this experiment, the storage modulus representing properties of a solid should be larger than the loss modulus representing properties of a liquid, and the smaller the tan δ value of the loss modulus to the storage modulus, the greater the ability to maintain the fixed state.

The high shear stress condition (25 Pa) is intended to confirm flowability of the CNFs when injecting the facial filler into the skin. In this experiment, the loss modulus representing the properties of the liquid should be larger than the storage modulus representing the properties of the solid, and the larger the tan δ value of the loss modulus to the storage modulus, the greater the exhibited flowability.

In this process, in order to determine the rheology modifier of CNFs under the low shear stress of 0.1 Pa, which is the skin injection condition, the storage modulus as the storage coefficient and the loss modulus as the loss coefficient of CNFs were confirmed.

Herein, the tan δ value was calculated by Equation 2 below. The tan δ value is a numerical value indicating whether a substance is closer to the solid or liquid. A value closer to 1 indicates a solution state (low elasticity), and a value closer to 0 may be defined as an elastic body with high elasticity.

[Equation 2]

Tan δ (tangent delta) = Loss modulus / Storage modulus

The tan δ value of Equation 2 is defined as the tan δ value of the loss elastic modulus to the storage elastic modulus.

The storage modulus and the loss modulus were respectively measured for each substitution degree of the carboxymethyl group with respect to the CNFs by the above method, and measured results were compared with hyaluronic acid (HA) under the same conditions. Results thereof are shown in Tables 5 and 6 below.

Table 5 below shows the measured results at the low shear stress of 0.1 Pa, which is a condition after skin injection, wherein the storage coefficient refers to the storage modulus.

[TABLE 5]

| Substitution degree of CNF | 0.2 | 0.3 | 0.5 | 0.6 | 0.7 | HA |
|---|---|---|---|---|---|---|
| Storage modulus (storage coefficient) (Pa) | 558 | 766 | 979 | 1,029 | 353 | 47 |
| Loss modulus (Pa) | 84 | 114 | 149 | 143 | 125 | 33 |
| tan δ | 0.14 | 0.15 | 0.15 | 0.14 | 0.36 | 0.71 |

Table 6 below shows the measured results at the high shear stress of 25 Pa, which is a condition before skin injection, wherein the loss coefficient refers to the loss modulus.

[TABLE 6]

| Substitution degree of CNF | 0.2 | 0.3 | 0.5 | 0.6 | 0.7 | HA |
|---|---|---|---|---|---|---|
| Storage modulus (Pa) | 64 | 229 | 249 | 235 | 29 | 42 |
| Loss modulus (loss coefficient) (Pa) | 94 | 249 | 284 | 306 | 71 | 32 |
| tan δ | 1.47 | 1.09 | 1.14 | 1.30 | 2.44 | 0.77 |

Referring to Tables 5 and 6 above, it can be deduced from the data that the CNF sample is in a liquid state upon elution of the sample from a syringe, and is in a solid state with high viscosity after injection.

From the above experimental results, it can be confirmed that the prepared CNFs have superior physical properties compared to hyaluronic acid within a range of substitution degree of 0.3 to 0.6 in both the low shear stress and high shear stress. In particular, having a high loss modulus compared to the storage modulus at the high shear stress, which is the state before injection, means that the CNFs maintain a liquid state at high pressure.

The tan δ values of the loss modulus for the storage modulus measured at the low shear stress and the high shear stress according to the above results were compared with that of hyaluronic acid (HA), and the results are shown in the graphs of FIGS. 6A and 6B. From these results, it was confirmed that the CNFs had better physical properties

than that of hyaluronic acid within the range of the substitution degree of 0.3 and 0.6.

[Experiment 5] Cytotoxicity test

A cytotoxicity test was conducted on the prepared CNFs using a CCk8 kit, and this experiment was performed according to a procedure of measuring an amount of color developed by reducing a reagent called CCk-8 by cell dehydrogenase as absorbance.

Toxicity comparison of the CNFs with hyaluronic acid (HA) was performed for each substitution degree of 0.2 to 0.7.

The experiment was conducted by controlling the concentrations of each sample to 0.1%, 1.0%, 10% and 50% in total media.

These experimental results are shown in FIGS. 7A and 7B, which are results obtained from a comparative experiment when each concentration was injected to kidney cells (HEK293) and hepatocytes (HepG2) under the same conditions. Since the toxicity level at each concentration was insignificant when comparing with hyaluronic acid, it was confirmed that the CNFs had no toxicity problem compared to hyaluronic acid.

[Industrial Applicability]

The modified cellulose in the cellulose nanofibril form with a thin thickness and short length prepared according to the present invention has physical properties such as water retention value, storage modulus, and loss modulus, etc., which are superior to hyaluronic acid, as well as excellent moisturizing power as described above, and may be used as a dermal filler composition and a skin beauty enhancing composition by replacing hyaluronic acid.

In addition, since the modified cellulose of the present invention having specific physical properties has greatly improved water holding power and moisturizing power, it is expected that application thereof will be greatly increased in related industries such as cosmetics, food, medicine, and the like in the future.

## Claims

1. A dermal filler composition comprising: modified cellulose in a cellulose nanofibril form as an effective ingredient, wherein the modified cellulose is made of pulp cellulose, contains a carboxymethyl group as a substituent, and has an average diameter of 3.0 to 10.0 nm and a length of 100 to 560 nm.

2. The dermal filler composition according to claim 1, wherein the modified cellulose contains a substituent substituted with a carboxymethyl group and has a water retention value (WRV) of 200 to 450%, which is obtained by measuring a water content after centrifugation for 50 minutes under 968G (gravitational acceleration) conditions.

3. The dermal filler composition according to claim 1, wherein the modified cellulose has a viscosity of 3,000 to 10,000 cps.

4. The dermal filler composition according to claim 1, wherein the modified cellulose has a tan δ value of a loss modulus to a storage modulus in a range of 0.1 to 0.3 under a low shear stress condition of 0.1 Pa, and a tan δ value of the loss modulus to the storage modulus in a range of 1.0 to 2.0 under a high shear stress condition of 25 Pa.

5. The dermal filler composition according to claim 1, wherein the modified cellulose has a storage modulus of 600 to 1,200 Pa, and a loss modulus of 150 to 400 Pa.

6. The dermal filler composition according to claim 1, wherein the modified cellulose has a substitution degree of a carboxymethyl group of 0.3 to 0.6.

7. The dermal filler composition according to claim 1, wherein the modified cellulose has: a water retention value (WRV) of 200 to 450%, which is obtained by measuring a water content after centrifugation for 50 minutes under 968G (gravitational acceleration) conditions; and a viscosity of 3,000 to 10,000 cps.

8. The dermal filler composition according to claim 1, wherein the modified cellulose has: a water retention value (WRV) of 200 to 450%, which is obtained by measuring a water content after centrifugation for 50 minutes under 968G (gravitational acceleration) conditions; a viscosity of 3,000 to 10,000 cps; and a tan δ value of a loss modulus to a storage modulus in a range of 0.1 to 0.3 under a low shear stress condition of 0.1 Pa, and a tan δ value of the loss

modulus to the storage modulus in a range of 1.0 to 2.0 under a high shear stress condition of 25 Pa.

**9.** The dermal filler composition according to claim 1, wherein the modified cellulose has: a water retention value (WRV) of 200 to 450%, which is obtained by measuring a water content after centrifugation for 50 minutes under 968G (gravitational acceleration) conditions; a viscosity of 3,000 to 10,000 cps; a tan $\delta$ value of a loss modulus to a storage modulus in a range of 0.1 to 0.3 under a low shear stress condition of 0.1 Pa, and a tan $\delta$ value of the loss modulus to the storage modulus in a range of 1.0 to 2.0 under a high shear stress condition of 25 Pa; and a substitution degree of a carboxymethyl group of 0.3 to 0.6.

**10.** The dermal filler composition according to claim 1, wherein the modified cellulose has: a viscosity of 3,000 to 10,000 cps; a tan $\delta$ value of a loss modulus to a storage modulus in a range of 0.1 to 0.3 under a low shear stress condition of 0.1 Pa, and a tan $\delta$ value of the loss modulus to the storage modulus in a range of 1.0 to 2.0 under a high shear stress condition of 25 Pa; and a substitution degree of a carboxymethyl group of 0.3 to 0.6.

**11.** The dermal filler composition according to claim 1, wherein the modified cellulose has: a tan $\delta$ value of a loss modulus to a storage modulus in a range of 0.1 to 0.3 under a low shear stress condition of 0.1 Pa, and a tan $\delta$ value of the loss modulus to the storage modulus in a range of 1.0 to 2.0 under a high shear stress condition of 25 Pa; and a substitution degree of a carboxymethyl group of 0.3 to 0.6.

**12.** The dermal filler composition according to claim 1, wherein the modified cellulose has: a storage modulus of 600 to 1,200 Pa, and a loss modulus of 150 to 400 Pa; and a substitution degree of a carboxymethyl group of 0.3 to 0.6.

**13.** The dermal filler composition according to claim 1, wherein the modified cellulose has: a water retention value (WRV) of 200 to 450%, which is obtained by measuring a water content after centrifugation for 50 minutes under 968G (gravitational acceleration) conditions; a tan $\delta$ value of a loss modulus to a storage modulus in a range of 0.1 to 0.3 under a low shear stress condition of 0.1 Pa, and a tan $\delta$ value of the loss modulus to the storage modulus in a range of 1.0 to 2.0 under a high shear stress condition of 25 Pa; and a substitution degree of a carboxymethyl group of 0.3 to 0.6.

**14.** The dermal filler composition according to claim 1, wherein the modified cellulose has: a water retention value (WRV) of 200 to 450%; and a substitution degree of carboxymethyl group of 0.3 to 0.6.

**15.** The dermal filler composition according to claim 1, wherein the modified cellulose has: a tan $\delta$ value of a loss modulus to a storage modulus in a range of 0.1 to 0.3 under a low shear stress condition of 0.1 Pa, and a tan $\delta$ value of the loss modulus to the storage modulus in a range of 1.0 to 2.0 under a high shear stress condition of 25 Pa; and a storage modulus of 600 to 1,200 Pa, and a loss modulus of 150 to 400 Pa.

**16.** The dermal filler composition according to claim 1, wherein the modified cellulose has: a tan $\delta$ value of a loss modulus to a storage modulus in a range of 0.1 to 0.3 under a low shear stress condition of 0.1 Pa, and a tan $\delta$ value of the loss modulus to the storage modulus in a range of 1.0 to 2.0 under a high shear stress condition of 25 Pa; a storage modulus of 600 to 1,200 Pa, and a loss modulus of 150 to 400 Pa; and a substitution degree of a carboxymethyl group of 0.3 to 0.6.

**17.** The dermal filler composition according to any one of claims 1 to 16, the dermal filler composition is formed in a hydrogel form.

**18.** A facial filler comprising the dermal filler composition according to any one of claims 1 to 16 as an effective ingredient.

**19.** A skin beauty enhancing composition comprising: modified cellulose in a cellulose nanofibril form as an effective ingredient, wherein the modified cellulose is made of pulp cellulose, contains a carboxymethyl group as a substituent, and has: an average diameter of 3.0 to 10.0 nm and a length of 100 to 560 nm; and a water retention value (WRV) of 200 to 450%, which is obtained by measuring a water content after centrifugation for 50 minutes under 968G (gravitational acceleration) conditions.

**20.** A skin beauty enhancing composition comprising: modified cellulose in a cellulose nanofibril form as an effective ingredient, wherein the modified cellulose is made of pulp cellulose, contains a carboxymethyl group as a substituent, and has: an average diameter of 3.0 to 10.0 nm and a length of 100 to 560 nm; and a tan $\delta$ value of a loss modulus to a storage modulus in a range of 0.1 to 0.3 under a low shear stress condition of 0.1 Pa, and a tan $\delta$ value of the

loss modulus to the storage modulus in a range of 1.0 to 2.0 under a high shear stress condition of 25 Pa.

[FIG. 1]

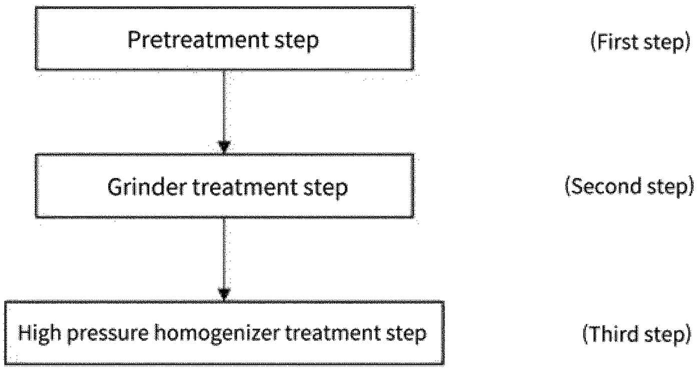


[FIG. 2]

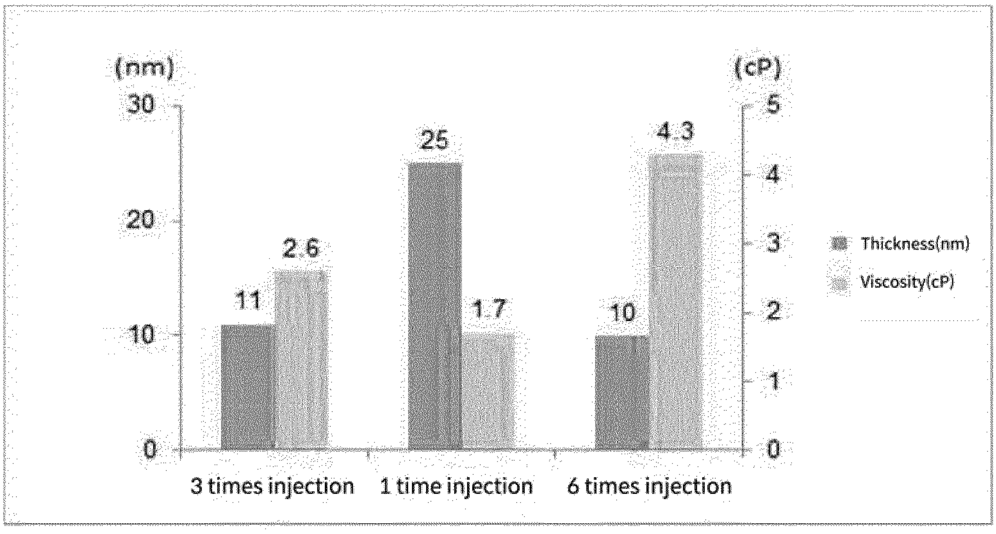


[FIG. 3]

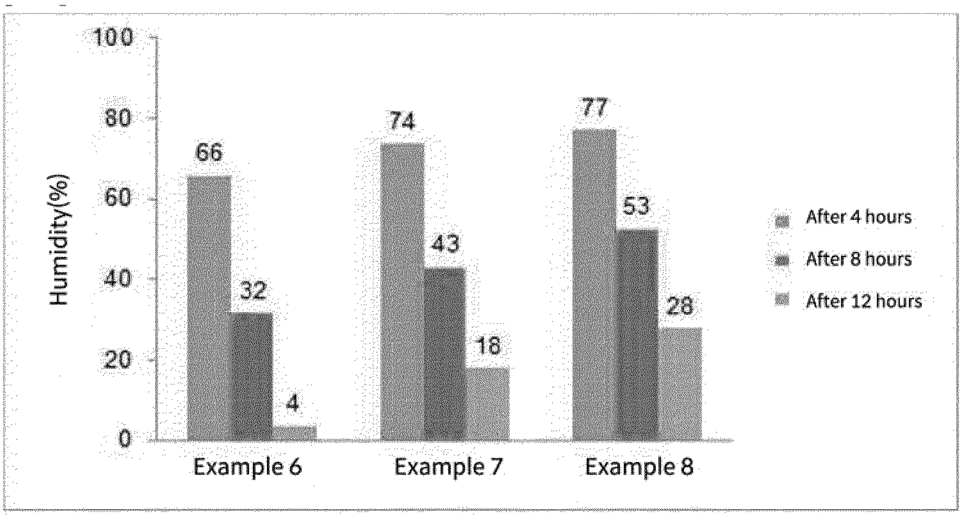

[FIG. 4]

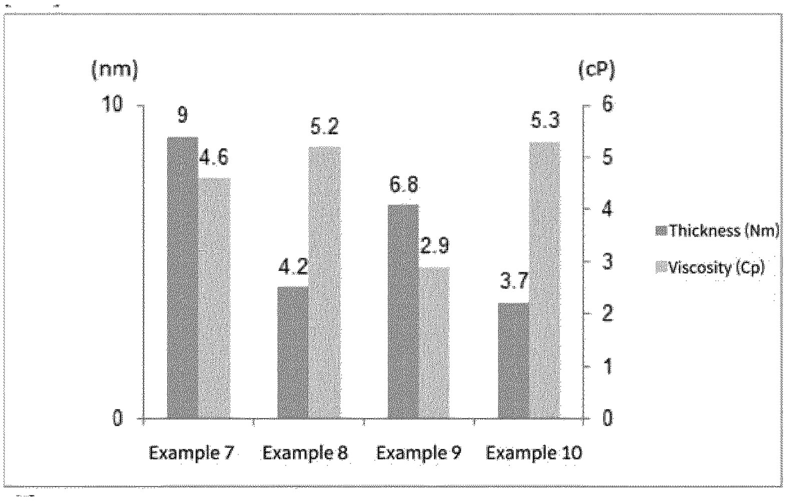


[FIG. 5]

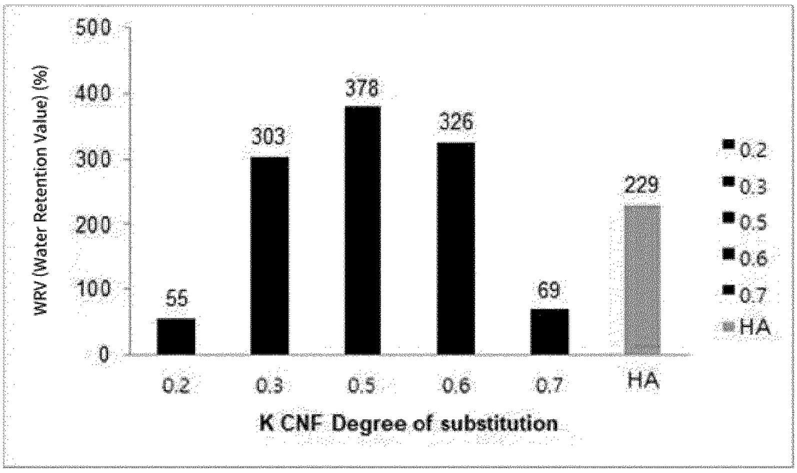


[FIG. 6A]

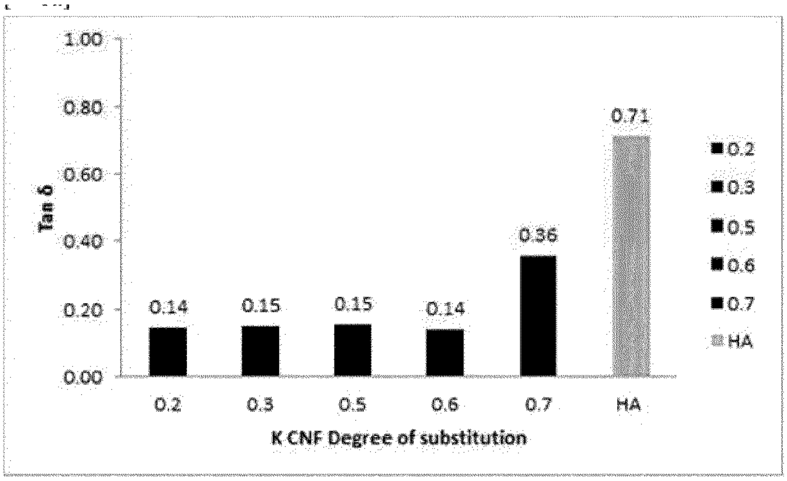

[FIG. 6B]

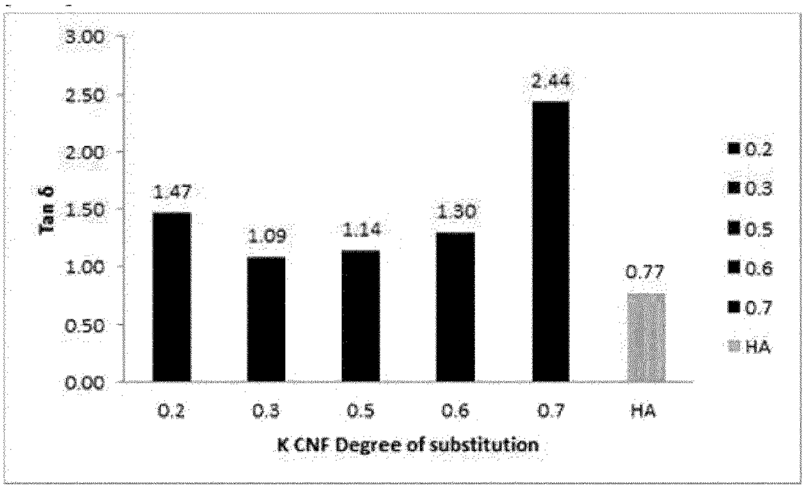


[FIG. 7A]

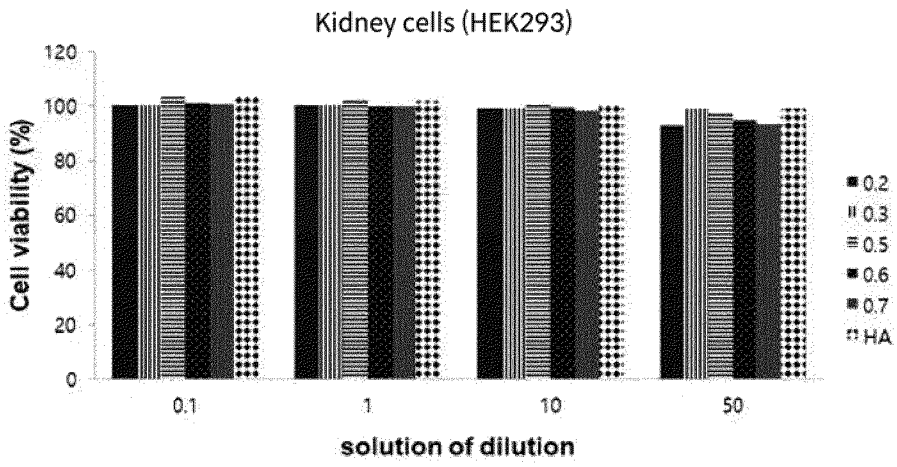


[FIG. 7B]

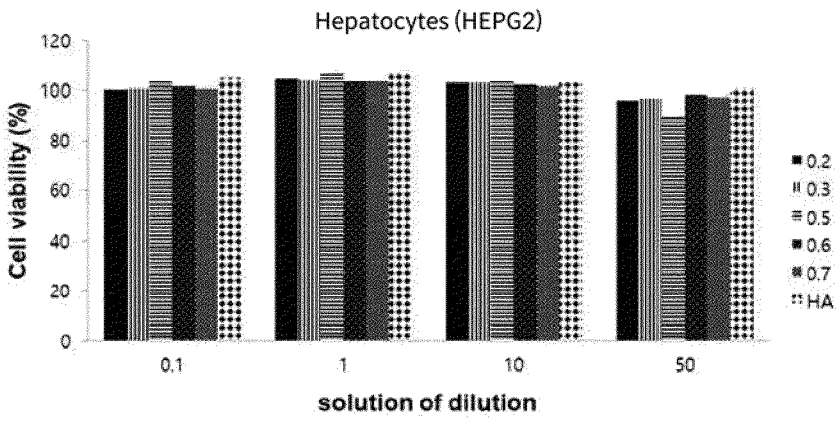

INTERNATIONAL SEARCH REPORT

International application No. PCT/KR2019/007709

A. CLASSIFICATION OF SUBJECT MATTER

*A61L 27/20(2006.01)i, A61L 27/52(2006.01)i, A61F 2/00(2006.01)i, A61K 8/73(2006.01)i, A61K 8/02(2006.01)i, A61Q 19/00(2006.01)i*

According to International Patent Classification (IPC) or to both national classification and IPC

B. FIELDS SEARCHED

Minimum documentation searched (classification system followed by classification symbols)

A61L 27/20; A61K 8/02; A61K 8/73; A61L 27/52; A61L 27/58; B29C 47/00; C08B 1/00; D01F 2/00; D21C 9/00; D21H 11/18; A61F 2/00; A61Q 19/00

Documentation searched other than minimum documentation to the extent that such documents are included in the fields searched

Korean utility models and applications for utility models: IPC as above

Japanese utility models and applications for utility models: IPC as above

Electronic data base consulted during the international search (name of data base and, where practicable, search terms used)

eKOMPASS (KIPO internal) & Keywords: denaturalized cellulose, pulp, filler, fibril

C. DOCUMENTS CONSIDERED TO BE RELEVANT

| Category* | Citation of document, with indication, where appropriate, of the relevant passages | Relevant to claim No. |
|---|---|---|
| Y | KR 10-2019-0062101 A (SEOUL NATIONAL UNIVERSITY R&DB FOUNDATION) 05 June 2019<br>See claims 1-12. | 1-20 |
| Y | KR 10-2017-0117368 A (MERZ PHARMA GMBH. & CO. KGAA.) 23 October 2017<br>See claim 1. | 1-20 |
| A | JP 2013-526657 A (FPINNOVATIONS) 24 June 2013<br>See the entire document. | 1-20 |
| A | KR 10-2018-0085005 A (JENACELL GMBH.) 25 July 2018<br>See the entire document. | 1-20 |
| A | KR 10-2018-0094948 A (FINECELL SWEDEN AB.) 24 August 2018<br>See the entire document. | 1-20 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 23 MARCH 2020 (23.03.2020) | **23 MARCH 2020 (23.03.2020)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**
Information on patent family members

International application No.
**PCT/KR2019/007709**

| Patent document cited in search report | Publication date | Patent family member | Publication date |
|---|---|---|---|
| KR 10-2019-0062101 A | 05/06/2019 | None | |
| KR 10-2017-0117368 A | 23/10/2017 | CN 106999625 A | 01/08/2017 |
| | | EP 3218023 A1 | 20/09/2017 |
| | | EP 3218023 B1 | 05/09/2018 |
| | | JP 2017-533785 A | 16/11/2017 |
| | | US 10335512 B2 | 02/07/2019 |
| | | US 2017-0333596 A1 | 23/11/2017 |
| | | WO 2016-074794 A1 | 19/05/2016 |
| JP 2013-526657 A | 24/06/2013 | CN 103038402 A | 10/04/2013 |
| | | CN 103038402 B | 15/07/2015 |
| | | CN 104894668 A | 09/09/2015 |
| | | CN 104894668 B | 12/04/2017 |
| | | EP 2569468 A1 | 20/03/2013 |
| | | EP 2569468 B1 | 25/01/2017 |
| | | EP 2569468 B2 | 18/12/2019 |
| | | JP 5848330 B2 | 27/01/2016 |
| | | US 2011-0277947 A1 | 17/11/2011 |
| | | US 9856607 B2 | 02/01/2018 |
| | | WO 2011-140643 A1 | 17/11/2011 |
| KR 10-2018-0085005 A | 25/07/2018 | CA 3005979 A1 | 01/06/2017 |
| | | CN 108697618 A | 23/10/2018 |
| | | EP 3380072 A1 | 03/10/2018 |
| | | JP 2019-503995 A | 14/02/2019 |
| | | US 2018-0303726 A1 | 25/10/2018 |
| | | WO 2017-089005 A1 | 01/06/2017 |
| KR 10-2018-0094948 A | 24/08/2018 | CA 3008365 A1 | 22/06/2017 |
| | | CN 108779183 A | 09/11/2018 |
| | | EP 3390457 A1 | 24/10/2018 |
| | | JP 2019-502798 A | 31/01/2019 |
| | | US 2018-0291119 A1 | 11/10/2018 |
| | | WO 2017-105337 A1 | 22/06/2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101487475
- KR 1020150110549
- KR 1020170117368
- US 9371402 B
- WO 2007014285 A